# EUROPEAN PATENT SPECIFICATION

(11) **EP 1 689 430 B1**
(45) Date of publication and mention of the grant of the patent: **29.07.2009**
(21) Application number: 04794411.1
(22) Date of filing: 07.10.2004
(51) Int. Cl.: A61K 39/00, C12N 9/02

(54) **TYROSINASE MUTANT AND METHODS OF USE THEREOF**
TYROSINASEMUTANTE UND VERFAHREN ZUR VERWENDUNG DAVON
MUTANT DE TYROSINASE ET PROCEDES D'UTILISATION CORRESPONDANTS

(30) Priority: 07.10.2003 US 508879 P
(43) Date of publication of application: 16.08.2006
(73) Proprietor: United Therapeutics Corporation, Washington, DC 20009 (US)
(72) Inventor: PETRESCU, Stefana M., Institute of Biochemistry, 77700 Bucharest 17 (RO); POPESCU, Costin I., Institute of Biochemistry, 77700 Bucharest 17 (RO); DWEK, Raymond A., Glycobiology Institute, Oxford OX1 3QU (GB)
(74) Representative: Warcoin, Jacques
(86) International application number: PCT/US2004/033048
(87) International publication number: WO 2005/035723

(56) References cited:
- SIMONOVA MARIA ET AL: "Tyrosinase mutants are capable of prodrug activation in transfected nonmelanotic cells" CANCER RESEARCH, vol. 60, no. 23, 1 December 2000 (2000-12-01), pages 6656-6662, XP002441618 ISSN: 0008-5472
- HALABAN RUTH ET AL: "Endoplasmic reticulum retention is a common defect associated with tyrosinase-negative albinism" PROCEEDINGS OF THE NATIONAL ACADEMY OF SCIENCES OF THE UNITED STATES OF AMERICA, vol. 97, no. 11, 23 May 2000 (2000-05-23), pages 5889-5894, XP002441619 ISSN: 0027-8424
- HALABAN RUTH ET AL: "Coexpression of wild-type tyrosinase enhances maturation of temperature-sensitive tyrosinase mutants" JOURNAL OF INVESTIGATIVE DERMATOLOGY, vol. 119, no. 2, August 2002 (2002-08), pages 481-488, XP002441620 ISSN: 0022-202X
- WOELFEL CATHERINE ET AL: "Transporter (TAP)- and proteasome-independent presentation of a melanoma-associated tyrosinase epitope" INTERNATIONAL JOURNAL OF CANCER, vol. 88, no. 3, 1 November 2000 (2000-11-01), pages 432-438, XP002441621 ISSN: 0020-7136
- HALABAN RUTH ET AL: "Abnormal acidification of melanoma cells induces tyrosinase retention in the early secretory pathway" JOURNAL OF BIOLOGICAL CHEMISTRY, vol. 277, no. 17, 26 April 2002 (2002-04-26), pages 14821-14828, XP002441622 ISSN: 0021-9258
- WANG YE ET AL: "Oligosaccharide trimming plays a role in the endoplasmic reticulum-associated degradation of tyrosinase" BIOCHEMICAL AND BIOPHYSICAL RESEARCH COMMUNICATIONS, vol. 271, no. 1, 29 April 2000 (2000-04-29), pages 22-27, XP002441623 ISSN: 0006-291X
- LE FUR NATHALIE ET AL: "Up-regulation of specific tyrosinase mRNAs in mouse melanomas with the c-2j gene substituted for the wild-type tyrosinase allele: Utilization in design of syngeneic immunotherapy models" PROCEEDINGS OF THE NATIONAL ACADEMY OF SCIENCES OF THE UNITED STATES OF AMERICA, vol. 94, no. 14, 1997, pages 7561-7565, XP002441624 ISSN: 0027-8424
- OLIVARES CONCEPCION ET AL: "Conformation-dependent post-translational glycosylation of tyrosinase. Requirement of a specific interaction involving the CuB metal binding site." JOURNAL OF BIOLOGICAL CHEMISTRY, vol. 278, no. 18, 2 May 2003 (2003-05-02), pages 15735-15743, XP002441625 ISSN: 0021-9258
- CHEN Y.T. ET AL: 'Immunophenotyping of melanomas for tyrosinase: Implications for vaccine development.' PROC NATL ACAD SCI USA. vol. 92, August 1995, pages 8125 - 8129, XP002992712
- TAKEDA A. ET AL: 'Molecular Basis of Tyrosinase-negative Oculocutaneous Albinism.' J BIOL CHEM. vol. 265, no. 29, 15 October 1990, pages 17792 - 17797, XP002992713
- ÚJVÁRI ET AL.: "Translation rate of human tyrosinase determines its N-linked glycosylation level" J. BIOL. CHEM., vol. 276, no. 8, 23 February 2001 (2001-02-23), pages 5924-5931,

## Description

### BACKGROUND OF THE INVENTION

### 1. Field of the Invention

The present invention relates to tyrosinase mutants of claims 1 and 25. In particular, the present invention describes tyrosinase mutants and their use for preparing a medicament for treating melanoma.

### 2. Background of the Invention

The incidence of malignant melanoma is increasing more rapidly than any other type of human cancer in North America (Armstrong et al. (1994) Cancer Surv. 19-20:219-240). Although melanoma is a curable cancer, the primary tumor must be removed at a very early stage of disease progression, i.e., before it has spread to distant sites. The presence of micrometastases can, and often do, lead to eventual symptomatic metastases. Thus, there is a need to devise a therapeutic method for treating melanoma.

Accordingly, the inventors investigated the folding pathway of the globular domain of tyrosinase in the presence and absence of the tyrosinase transmembrane domain. Tyrosinase (monophenol, 3,4-dihydroxyphenylalanine: oxygen oxidoreductase, EC 1. 14.18. 1) is a type I membrane glycoprotein whose maturation in the presence of the ER quality control has been well documented (Petrescu *et al.,* 2000; Halaban *et al.,* 1997; Toyofuku *et al.,* 2001; Branza-Nichita *et al.,* 2000). Tyrosinase is generally exclusive to pigment-producing cells (melanocytes) and is a differentiation antigen in melanoma. The present invention relates to tyrosinase mutants is retained in the ER. The mutants are degraded by proteasomes and presented on the cell surface by MHC class I molecules.

The tyrosinase mutant described by Simonova et al. (Cancer Research, 60:23, 6656-6662, 2000) is not retained within the ER but is instead secreted. Since this mutant is active, it can produce toxic-free radicals and can be used as a prodrug that activates itself and kills cells. This is in contrast to the mutant of the present invention, which is retained in the early secretory pathway and is inactive, unable to produce melanin and free radicals with toxic effects. The tyrosinase mutant of the present invention will activate the immune system and the immune system cells will in turn kill a melanoma cell, while avoiding any toxicity that a prodrug would produce.

Halaban et al. (PNAS, 97:11, 5889-5894, 2000) describes three tyrosinase mutants that are retained in the ER, this document does not describe a tyrosinase mutant that lacks its transmembrane domain and does not interact with calnexin.

Halaban et al. (J. Investigative Dermatology, 119:2, 481-488, 2002) describes single-site glycosylation deletion mutants of tyrosinase. This document does not describe a glycosylation mutant made by mutating Asn at position 86. Further, mutants lacking the transmembrane domain and which do not interact with calnexin are not disclosed.

These documents are silent with respect to the presentation of tyrosinase antigens for melanoma treatment.

Woelfel et al. (International J. of Cancer, 88:3, 432-438, 2000) describes the transporter and proteasome-independent presentation of tyrosinase peptide 1-9 by HLA-A2.1. The signal sequence derived peptide 1-9 is considered as a candidate for specific immunotherapy.

The present invention provides tyrosinase mutants having the ability to elicit an improved immune response for treating melanoma.

Folding of soluble and membrane-bound glycoprotein in eukaryotic cells begins while the nascent polypeptide chain is translocated into the ER lumen through the translocon pore (Hardesty *et al.,* 1999). The process continues post-translationally by repeated folding and refolding steps in the presence of the ER-resident chaperones and results in a product able to exit the ER (Trombetta and Helenius, 1998, Chen and Helenius, 2000). Misfolded and improperly assembled proteins are usually retro-translocated into the cytoplasm to be degraded by proteasomes (Brodsky, 1997).

The folding pathway of anchor-free (soluble) and membrane-bound proteins may differ substantially because of events related to the insertion of the transmembrane domain (TM) into the lipid bilayer. It is known that the translocon offers a protective and restrictive environment acting itself as a chaperone for the protein chain during translocation (Chen and Helenius, 2000). Recently, it has been shown that the TM is unable to integrate directly into the ER lipid bilayer (Mothes *et al.,* 1997). Instead, the TM domain is released into the aqueous channel upon synthesis and inserted into the lipid bilayer by lateral diffusion. The efficiency and speed by which the diffusion process occurs is dependent on the hydrophobicity of the TM domain (Heinrich *et al.,* 2000). For example, a nascent chain can be retained in the translocon for longer periods of time when the TM regions are less hydrophobic. Thus, time spent by the nascent chain inside or in the proximity of the translocon may be dependent on the amino acid composition of the TM region.

Based on these findings, the inventors theorized that the TM domain could act as a driving factor for events related to folding that occur during translocation. Folding of the nascent chain in the ER lumen is tightly regulated by a quality control based on the recognition of the monoglucosylated N-glycans by the lectin chaperones calnexin (CNX) and calreticulin (CRT) (Helenius and Aebi, 2001; Schrag *et al.,* 2001). While studies show that quality control also monitors the assembly of TM domains into the lipid bilayer (Cannon and Creswell, 2001), little is known on the role of the TM domain in the folding process of membrane proteins.

To further investigate the role of a TM domain, the present inventors constructed a human tyrosinase mutant whose trafficking is stopped at the ER level. In other words, the mutant tyrosinase is misfolded and is retained in the ER by a quality control system. Thus, the mutant tyrosinase is retro-translocated to proteasomes for degradation, and following degradation, the resultant peptides are presented on the cell surface by MHC class I molecules. As such, the mutant tyrosinase of the present invention can be used in melanoma immunotherapy as a vaccine drug designed to enhance the immune response of CTLs against melanoma cells.

An important aspect of the immune response, in particular as it relates to vaccine efficacy, is the manner in which antigen is processed so that it can be recognized by the specialized cells of the immune system. Distinct antigen processing and presentation pathways are utilized and therefore, cell surface presentation of a particular antigen by a MHC class II or class I molecule to a helper T lymphocyte or a cytotoxic T lymphocyte, respectively, is dependent on the antigen processing pathway.

One pathway is a cytosolic pathway, which processes endogenous antigens expressed inside a cell. The antigen is degraded by a specialized protease complex in the cytosol of the cell, and the resulting antigen peptides are transported into the endoplasmic reticulum. This results in antigen binding to MHC class I molecules. By cross-presentation, exogenous antigens can be processed in the cytoplasm of professional antigen-presenting cells and bind to MHC class I molecules.

An alternative pathway is an endoplasmic reticulum pathway, which bypasses the cytosol. In the endoplasmic reticulum, the antigen peptides bind to MHC class I molecules, which are then transported to the cell surface for presentation to cytotoxic T lymphocytes of the immune system. Several studies point to the crucial role of cytotoxic T cells in both production and eradication of cancer by the immune system (Byrne et al., J. Immunol. 51:682 (1984); McMichael et al., N. Engl. J. Med. 309:13 (1983)).

A third pathway is an endocytic pathway, occurring in professional antigen-presenting cells, which processes antigens that exist outside the cell which results in antigen binding to MHC class II molecules. Such antigens are taken into the cell by endocytosis, which brings antigens into endosomes and then to lysosomes. Subsequently, the antigen is degraded by proteases into antigen peptides that bind MHC class II molecules and then transported to the cell surface for presentation to helper T lymphocytes of the immune system.

### SUMMARY OF THE INVENTION

Contemplated in the present invention is a polypeptide comprising a tyrosinase mutant, wherein the tyrosinase mutant is capable of accumulating in the endoplasmic reticulum and
a) the tyrosinase mutant is enzymatically inactive and lacks a transmembrane domain, or
b) the tyrosinase mutant lacks a tyrosinase transmembrane domain but contains a transmembrane domain from a protein that is not tyrosinase, or
c) the Asn residue at position 86 is changed to Gln residue in the tyrosinase mutant.

### BRIEF DESCRIPTION OF THE DRAWINGS

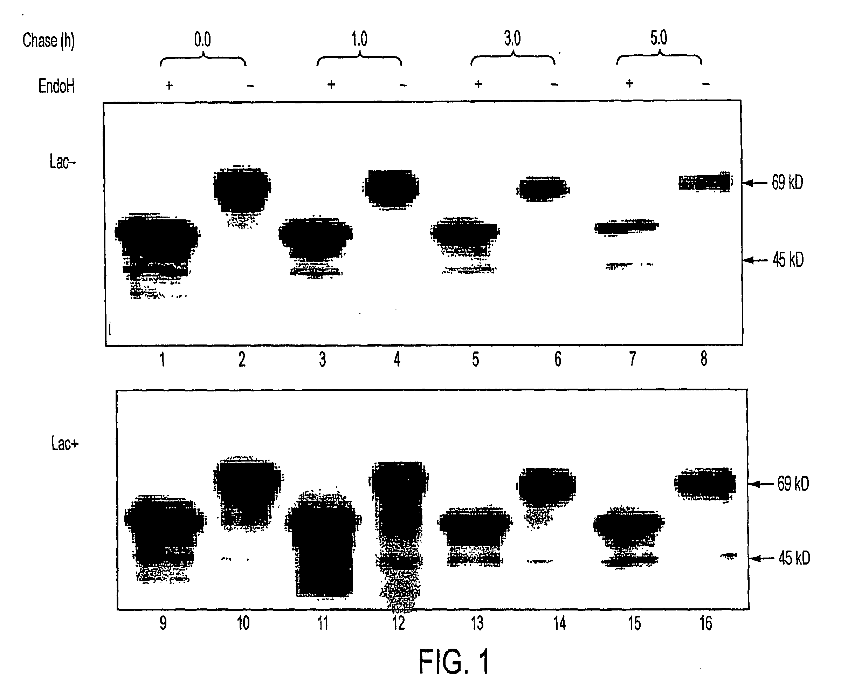

### Figure 1. Pulse-chase experiment showing that soluble tyrosinase is retained in the ER and degraded in proteasomes

CHO cells transfected with ST cDNA were pulsed for 20 min with [³⁵S] and chased in the absence (lanes 1-8) or presence (lanes 9-16) of 20 µM lactacystine for the indicated time period. Cell lysates were immunoprecipitated with T311 monoclonal antibodies and the immunoprecipitate samples were divided in half and digested with (+) or without (-) EndoH. The samples were run on a reducing 10% SDS-PAGE gel and visualized by autoradiography. The molecular mass marker is shown on the right side of the figure.

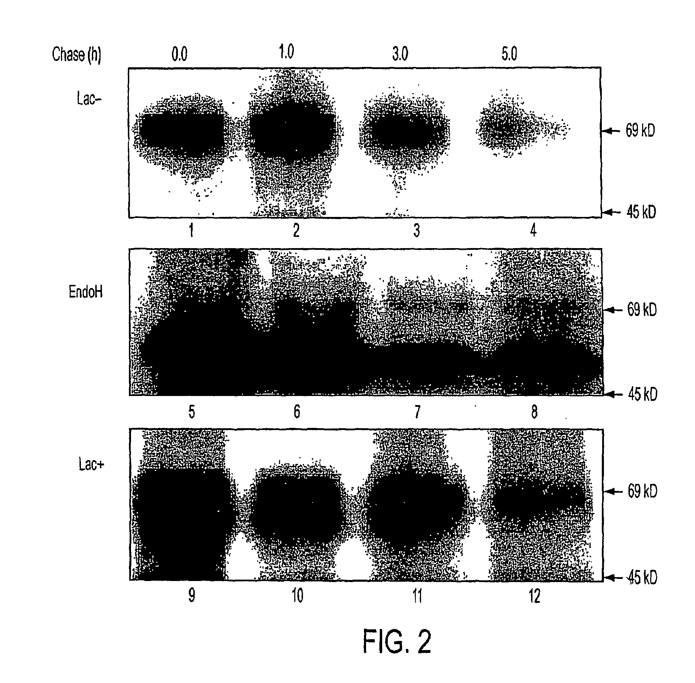

### Figure 2. Pulse-chase experiment indicating that wildtype (WT) tyrosinase is exported from the ER

WT transfected cells were pulsed for 20 min with [³⁵S] and chased in the absence (lanes 1-4) or presence (lanes 9-12) of 20 µM lactacystine for the indicated time period. Samples from lanes 5-8 were digested with EndoH. Cell lysates were immunoprecipitated with T311 antiserum. The samples were run on a 10 % SDS-PAGE gel and visualized by autoradiography.

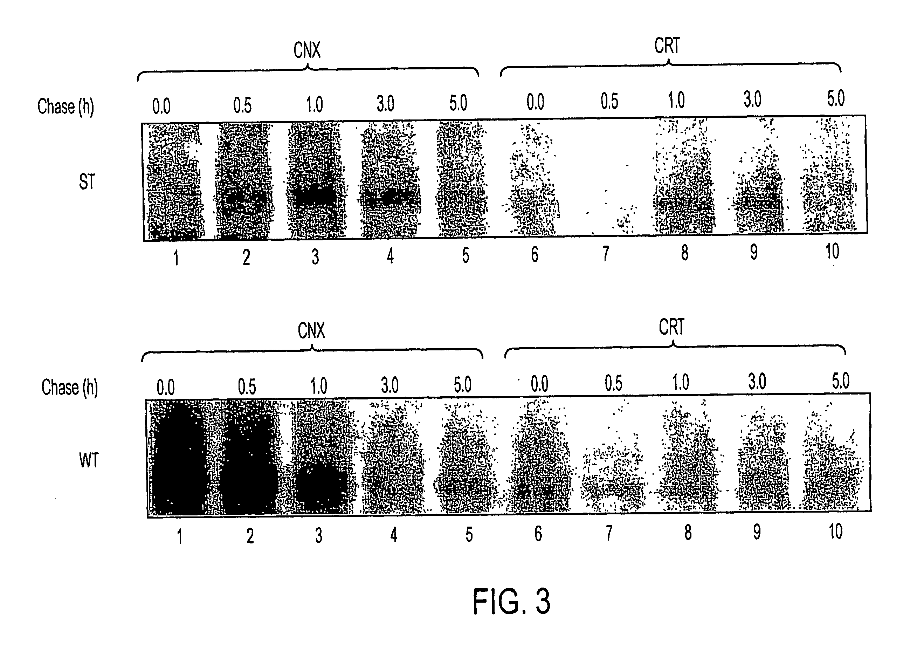

### Figure 3. Pulse-chase experiment showing the association of calnexin and calreticulin with WT and ST tyrosinase

CHO cells transfected with ST (lanes 1-10) or WT (lanes 11-20) were incubated in starvation buffer for 1h before a 20 min pulse with [³⁵S]. Cells were then chased for the indicated time period and cell lysates were immunoprecipitated with either an anti-calnexin antibody (CNX) or an anti-calreticulin antibody (CRT), followed by an anti-tyrosinase antibody (T311 antibody). The immunoprecipitates were run on a non- reducing 10% SDS-PAGE gel and visualized by autoradiography.

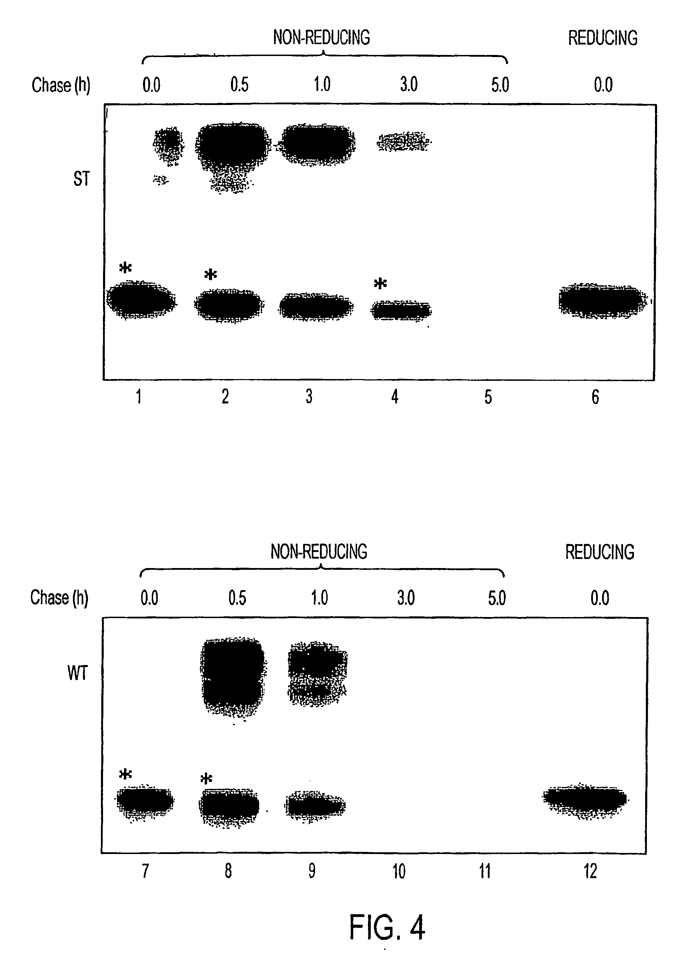

### Figure 4. Pulse-chase experiment demonstrating the folding pathway of soluble and wild type tyrosinase

CHO cells transfected with ST (lanes 1-6) or WT (lanes 7-11) tyrosinase were incubated in starvation buffer for 1h before a 20 min pulse with [³⁵S]. Cells were then chased for the indicated time period, and cell lysates were immunoprecipitated with an anti-tyrosinase antibody (T311 antibody). The immunoprecipitates were run on a non-reducing or reducing 10% SDS-PAGE gel and visualized by autoradiography.

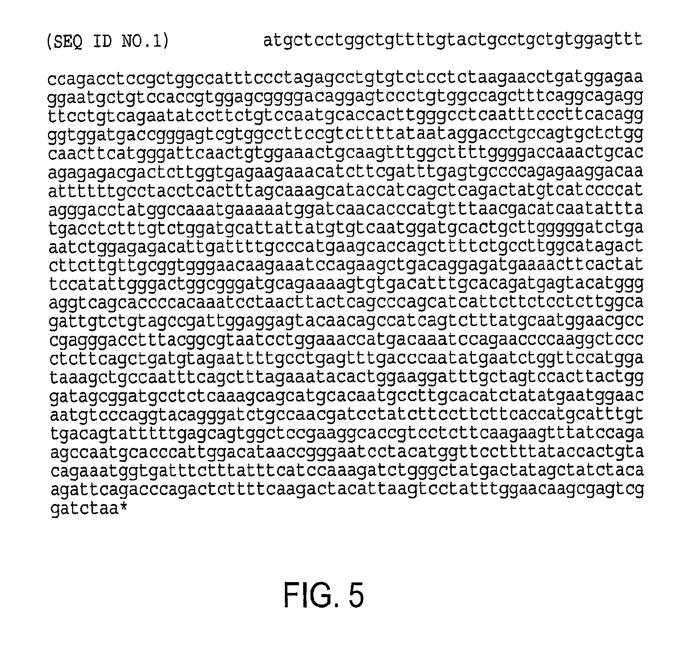

### Figure 5. Nucleic acid sequence of soluble tyrosinase (SEQ ID NO.1)

### Figure 6. Pulse-chase experiments showing that Tyrmut1 is retained in the ER.

Tyrmut1 transfected cells were pulsed for 20 minutes with [35S] and chased for 2 hours. Cell lysates were immunoprecipitated with T311 antiserum. The immunoprecipitates were divided in two and digested with (+) or without (-) EndoH. The samples were run on a 10% SDS-PAGE gel and visualized by autoradiography.

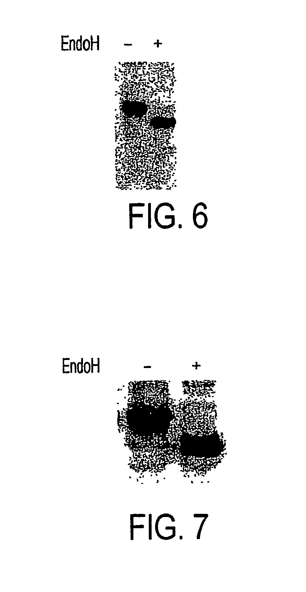

### Figure 7. Western blot experiment showing that Tyr_E2 chimera is retained in the ER.

Cells were treansfected with the Tyr_E2 construct and cell lystates were divided in two and digested with (+) or without (-) EndoH. The samples were run on a 10% SDS-PAGE gel, blotted and visualized by T311 antiserum by ECL chemiluminescence.

### DETAILED DESCRIPTION OF THE PREFERRED EMBODIMENTS

### Introduction

Human tyrosinase is a type I membrane glycoprotein and has 529 amino acids, seven occupied N-glycosylation sites, 17 cysteine residues grouped in two cysteine-rich domains, two copper binding domains and one C-terminal TM domain (Ujvari *et al.,* 2001). The inventors have constructed a truncated form of human tyrosinase which lacks a transmembrane (TM) domain. In the absence of the TM domain, the ER lumenal chain was unable to fold into a native conformation. However, productive folding of the truncated chain, yielding an active protein, was shown to occur when the translation rate slowed down. Enzymatically active soluble tyrosinase was produced at reduced temperatures also and productive folding was associated in both cases with CNX interaction in the early stages. This evidence supports a role for the TM domain in folding and maintaining the chain in the translocon environment, thereby facilitating its interaction with CNX.

Tyrosinase is constitutively expressed in melanoma cells generating tumoral antigens. Wild-type tyrosinase trafficks through the secretory pathway and targets melanosomes. The inventors constructed a human tyrosinase mutant whose trafficking is stopped at the endoplasmic reticulum (ER). The misfolded protein is then retained in the ER by a quality control system and retro-translocated to be degraded in proteasomes. Following cytoplasmic degradation, the resultant peptides are presented to cytotoxic T-lymphocytes (CTLs) by MHC class I molecules. As such, the mutant tyrosinase of the present invention can be used for preparing a medicament for melanoma immunotherapy as a vaccine drug designed to enhance the immune response of CTLs against melanoma cells.

While tyrosinase is expressed in normal melanocytes, melanoma cells, and retinal pigmented epithelial cells (RPE), a vaccine delivering a nucleic acid encoding the mutant tyrosinase of the present invention is nevertheless suitable for treating a melanoma. Therefore, while the vaccine drug may target both normal and abnormal melanocytes, humans can survive without melanocytes (Marks et al., Immunologic Research, 27, 409-425 (2003)). For example, vaccination may result in a condition known as vitiligo, a skin pigmentation disorder that does not pose a serious health concern.

A melanoma antigen or immunogen as described herein connotes a soluble tyrosinase mutant or fragment thereof that is capable of causing a cytotoxic T cell immune response in a patient such as a human or other mammal. Preferably, the soluble tyrosinase mutant is retained in the ER and lacks a transmembrane domain.

The term melanoma includes, but is not limited to, melanomas, metastatic melanomas, melanomas derived from either melanocytes or melanocytes related nevus cells, melanocarcinomas, melanoepitheliomas, melanosarcomas, melanoma in situ, superficial spreading melanoma, nodular melanoma, lentigo maligna melanoma, acral lentiginous melanoma, invasive melanoma or familial atypical mole and melanoma syndrome.

### Composition

Contemplated in the present invention is an immunogenic composition comprising a polynucleotide encoding a soluble tyrosinase mutant that is suitable for eliciting a CTL immune response and optionally, a pharmaceutically suitable excipient. Following delivery of the immunogenic composition to a target cell, the expressed tyrosinase of the present invention is retained in the endoplasmic reticulum, degraded, and then presented on the cell surface by MHC class I for antigen presentation. Preferably, the soluble tyrosinase mutant lacks a transmembrane domain. Most preferably, the soluble tyrosinase mutant comprises a nucleic acid sequence described in SEQ ID NO. 1 or variants thereof.

Also described in the present invention is a tyrosinase mutant that lacks one or more glycosylation sites. Such mutants are expected to be retained in the ER and degraded by endoplasmic reticulum associated degradation (ERAD) because they will be unable to interact with calnexin (which binds glycans) and yield misfolded polypeptides. These glycosylation mutants may or may not include a transmembrane domain. Other suitable tyrosinase mutants can be obtained by deletions or insertions into the tyrosinase cDNA sequence so long as they are able to induce ERAD.

For example, a tyrosinase mutant lacking one glycan in position 86 (Tyrmut1) is retained in the ER. Indeed, tyrosinase depends on calnexin interaction with glycans for correct folding and therefore, preventing glycan attachment at specific residues can cause misfolding and ER retention.

Likewise, albinism is regarded as a disease of tyrosinase misfolding and therefore, the tyrosinase expressed by a person with this disease is retained in the ER. Since the incidence of melanoma is low in albinos, this suggests that the tyrosinase mutants presented in the context of HLA complex break tolerance against tyrosinase antigens that are presented by melanoma cells.

Other tyrosinase mutants can be anchored by a transmembrane domain of another protein that contains an ER retention signal. Such a chimeric tyrosinase mutant results in a protein with an ER retention profile. These mutants may also additionally lack at least one glycosylation site.

The present invention also describes a nucleic acid sequence which encodes a novel melanoma antigen recognized by T cells. The melanoma antigen disclosed herein is a soluble mutant tyrosinase or fragment thereof that preferably is retained in the ER and lacks a transmembrane domain. Preferably, the nucleic acid sequence comprises the sequence described in SEQ ID NO. 1.

Also disclosed herein is a melanoma vaccine comprising a nucleic acid sequence encoding the tyrosinase mutant of the present invention, or fragment thereof, or a vaccine comprising a soluble tyrosinase mutant of the present invention or an immunogenic peptide derived from the tyrosinase mutant, for use in treating a melanoma. Also, the vaccine of the present invention may be administered in a pharmaceutically acceptable carrier. Pharmaceutically acceptable carriers typically include carriers known to those skilled in the art, including pharmaceutical adjuvants. Generally, these pharmaceutically acceptable carriers will include water, saline, buffers, and other compounds described, e.g., in the MERCK INDEX, Merck & Co., Rahway, N.J. See also Bioreversible Carriers in Drug Design, Theory and Application, Roche (ed.), Pergamon Press, (1987). Various considerations are described, e.g., in Gilman et al. (eds) (1990) Goodman and Gilman's: The Pharmacological Bases of Therapeutics, 8th Ed., Pergamon Press; Novel Drug Delivery Systems, 2nd Ed., Norris (ed.) Marcel Dekker Inc. (1989), and Remington's Pharmaceutical Sciences, the full disclosures of which are incorporated herein by reference.

The vaccine formulations described herein may be first evaluated in animal models or in nonhuman primates before humans. Conventional methods would be used to evaluate the immune response of the patient to determine the efficacy of the vaccine.

The present invention also contemplates variants of the polynucleotides and polypeptides described in the instant invention. In one embodiment, variants of the polynucleotide disclosed in SEQ ID NO. 1 are contemplated for use in the present invention. A "variant," as used herein, is understood to mean a nucleotide or amino acid sequence that deviates from the standard, or given, nucleotide or amino acid sequence of a particular gene or protein. The terms, "isoform," "isotype," and "analog" also refer to "variant" forms of a nucleotide or an amino acid sequence. An amino acid sequence that is altered by the addition, removal or substitution of one or more amino acids, or a change in nucleotide sequence, may be considered a "variant" sequence. The variant may have "conservative" changes, wherein a substituted amino acid has similar structural or chemical properties, *e.g.*, replacement of leucine with isoleucine. A variant may have "nonconservative" changes, *e.g.*, replacement of a glycine with a tryptophan. Analogous minor variations may also include amino acid deletions or insertions, or both. Guidance in determining which amino acid residues may be substituted, inserted, or deleted may be found using computer programs well known in the art such as Vector NTI Suite (InforMax, MD) software.

The conservative variants according to the invention generally preserve the overall molecular structure of the tyrosinase mutant. Given the properties of the individual amino acids comprising the disclosed tyrosinase mutant, some rational substitutions will be apparent. Amino acid substitutions, *i.e.* "conservative substitutions," may be made, for instance, on the basis of similarity in polarity, charge, solubility, hydrophobicity, hydrophilicity, and/or the amphipathic nature of the residues involved.

For example: (a) nonpolar (hydrophobic) amino acids include alanine, leucine, isoleucine, valine, proline, phenylalanine, tryptophan, and methionine; (b) polar neutral amino acids include glycine, serine, threonine, cysteine, tyrosine, asparagine, and glutamine; (c) positively charged (basic) amino acids include arginine, lysine, and histidine; and (d) negatively charged (acidic) amino acids include aspartic acid and glutamic acid. Substitutions typically may be made within groups (a)-(d). In addition, glycine and proline may be substituted for one another based on their ability to disrupt α-helices. Similarly, certain amino acids, such as alanine, cysteine, leucine, methionine, glutamic acid, glutamine, histidine and lysine are more commonly found in α-helices, while valine, isoleucine, phenylalanine, tyrosine, tryptophan and threonine are more commonly found in β-pleated sheets. Glycine, serine, aspartic acid, asparagine, and proline are commonly found in turns. Some preferred substitutions may be made among the following groups: (i) S and T; (ii) P and G; and (iii) A, V, L and I. Given the known genetic code, and recombinant and synthetic DNA techniques, the skilled scientist readily can construct DNAs encoding the conservative amino acid variants.

"Variant" may also refer to a "shuffled gene" such as those described in Maxygen-assigned patents. For instance, a variant of the present invention may include variants of sequences and desired polynucleotides that are modified according to the methods and rationale disclosed in U.S. 6,132,970, which is incorporated herein by reference in its entirety.

Likewise, the polynucleotide and polypeptide variants disclosed in the instant invention include polynucleotides and polypeptides that have at least 65%, at least 70%, at least 75%, at least 80%, at least 85%, at least 90%, at least 95% or at least 99% sequence identity to a nucleic acid encoding a soluble tyrosinase mutant or fragment thereof, or a soluble tyrosinase mutant polypeptide or fragment thereof, respectively, or hybridize under low, moderate or high stringent conditions to a nucleic acid encoding a soluble tyrosinase mutant or fragment thereof. Hybridization methods are well known to those skilled in the art. (See, *e.g.*, Ausubel, et al. (1997) Short Protocols in Molecular Biology, John Wiley & Sons, New York N.Y., Units 2.8-2.11, 3.18-3.19 and 4-6-4.9.) Conditions can be selected for hybridization where completely complementary probe and target can hybridize, i.e., each base pair must interact with its complementary base pair. Alternatively, conditions can be selected where probe and target have mismatches of up to about 10% but are still able to hybridize. Suitable conditions can be selected, for example, by varying the concentrations of salt in the prehybridization, hybridization, and wash solutions or by varying the hybridization and wash temperatures. With some substrates, the temperature can be decreased by adding formamide to the prehybridization and hybridization solutions. Hybridization can be performed at low stringency with buffers, such as 5xSSC with 1% sodium dodecyl sulfate (SDS) at 60°C., which permits hybridization between probe and target sequences that contain some mismatches to form probe/target complexes. Subsequent washes are performed at higher stringency with buffers such as 0.2xSSC with 0.1% SDS at either 45°C. (medium stringency) or 68°C. (high stringency), to maintain hybridization of only those probe/target complexes that contain completely complementary sequences. Background signals can be reduced by the use of detergents such as SDS, Sarcosyl, or Triton X-100, or a blocking agent, such as salmon sperm DNA.

The vaccines and immunogenic compositions for use in accordance with the present invention may optionally be formulated in conventional manner using one or more physiologically acceptable carriers or excipients. Thus, the may be formulated for administration by inhalation or insufflation (either through the mouth or the nose) or oral, buccal, parenteral or rectal administration. In a preferred embodiment, the pharmaceutical composition is prepared for parenteral administration.

For oral administration, the compositions of the present invention may take the form of, for example, tablets or capsules prepared by conventional means with pharmaceutically acceptable excipients such as binding agents (*e.g.*, pregelatinized maize starch, polyvinylpyrrolidone or hydroxypropyl methylcellulose); fillers (*e.g.*, lactose, microcrystalline cellulose or calcium hydrogen phosphate); lubricants (*e.g.*, magnesium stearate, talc or silica); disintegrants (*e.g.*, potato starch or sodium starch glycolate); or wetting agents (*e.g.*, sodium lauryl sulphate). The tablets may be coated by methods well known in the art. Liquid preparations for oral administration may take the form of, for example, solutions, syrups or suspensions, or they maybe presented as a dry product for constitution with water or other suitable vehicle before use. Such liquid preparations may be prepared by conventional means with pharmaceutically acceptable additives such as suspending agents (*e.g.*, sorbitol syrup, cellulose derivatives or hydrogenated edible fats); emulsifying agents (*e.g.*, lecithin or acacia); non-aqueous vehicles (*e.g.*, almond oil, oily esters, ethyl alcohol or fractionated vegetable oils); and preservatives (*e.g.*, methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the composition may take the form of tablets or lozenges formulated in conventional manner.

For administration by inhalation, the tyrosinase mutants according to the present invention are conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.* gelatin for use in an inhaler or insufflator may be formulated containing a powder mix of the compound and a suitable powder base such as lactose or starch.

As stated above, the soluble tyrosinase mutants of the present invention are preferably formulated for parenteral administration by injection, *e.g.*, by bolus injection or continuous infusion. Formulations for injection may be presented in unit dosage form, *e.g.*, in ampules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g.*, sterile pyrogen-free water, before use.

The compounds may also be formulated in rectal compositions such as suppositories or retention enemas, *e.g.*, containing conventional suppository bases such as cocoa butter or other glycerides.

In addition to the formulations described previously, the tyrosinase mutants of the present invention may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

Also described in the present invention is a DNA construct comprising a nucleotide sequence of a soluble tyrosinase mutant protein of the present invention. In a preferred embodiment, the tyrosinase nucleotide sequence is the nucleic acid sequence set forth in SEQ ID NO. 1 or variant thereof.

Recombinant protein production is well known in the art and is outlined briefly below.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include *E. coli, Bacillus subtilis, Salmonella typhimurium* and various species within the genera Pseudomonas, Streptomyces, and Staphylococcus, although others may, also be employed as a matter of choice. In a preferred embodiment, the prokaryotic host is *E. coli.*

Bacterial vectors may be, for example, bacteriophage-, plasmid- or cosmid-based. These vectors can comprise a selectable marker and bacterial origin of replication derived from commercially available plasmids typically containing elements of the well known cloning vector pBR322 (ATCC 37017). Such commercial vectors include, for example, GEM 1 (Promega Biotec, Madison, WI, USA), pBs, phagescript, PsiX174, pBluescript SK, pBs KS, pNH8a, pNH16a, pNH18a, pNH46a (Stratagene); pTrc99A, pKK223-3, pKK233-3, pKK232-8, pDR540, and pRIT5 (Pharmacia). A preferred vector according to the invention is pTriex (Novagen).

These "backbone" sections are combined with an appropriate promoter and the structural sequence to be expressed. Bacterial promoters include lac, T3, T7, lambda P_{R} or P_{L}, trp, and ara. T7 is the preferred bacterial promoter.

Following transformation of a suitable host strain and growth of the host strain to an appropriate cell density, the selected promoter is derepressed/induced by appropriate means (*e.g.*, temperature shift or chemical induction) and cells are cultured for an additional period. Cells are typically harvested by centrifugation, disrupted by physical or chemical means, and the resulting crude extract retained for further purification.

Various mammalian cell culture systems can also be employed to express recombinant protein. Examples of mammalian expression systems include selected mouse L cells, such as thymidine kinase-negative (TK) and adenine phosphoribosul transferase-negative (APRT) cells. Other examples include the COS-7 lines of monkey kidney fibroblasts, described by Gluzman, Cell 23: 175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking non-transcribed sequences. DNA sequences derived from the SV40 viral genome, for example, SV40 origin, early promoter, enhancer, splice, and polyadenylation sites may be used to provide the required non-transcribed genetic elements.

Mammalian promoters include CMV immediate early, HSV thymidine kinase, early and late SV40, LTRs from retrovirus, and mouse metallothionein-I. Exemplary mammalian vectors include pWLneo, pSV2cat, pOG44, pXT1, pSG (Stratagene) pSVK3, pBPV, pMSG, and pSVL (Pharmacia). In a preferred embodiment, the mammalian expression vector is pTriex.

In mammalian host cells, a number of viral-based expression systems may be utilized. In cases where an adenovirus is used as an expression vector, the coding sequence of interest may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome *(e.g.,* region E1 or E3) will result in a recombinant virus that is viable and capable of expressing a target protein in infected hosts. (*E.g.,* See Logan et al., 1984, Proc. Natl. Acad. Sci. USA 81: 3655-3659).

The nucleic acid sequences of the present invention are also suitable for use as probes for detecting expression of tyrosinase in normal and diseased tissue. Therefore, another aspect of the present invention relates to a bioassay for detecting mRNA encoding tyrosinase in a biological sample comprising contacting the sample with the nucleic acid sequence under conditions permitting hybridization between the nucleic acid and sample mRNA, and then detecting the complexes.

Detection of complexes in the bioassay can also be carried out by a variety of techniques. Detection of complexes by signal amplification can be achieved by several conventional labelling techniques including radiolabels and enzymes (Sambrook et. al., (1989) in "Molecular Cloning, A Laboratory Manual", Cold Spring Harbor Press, Plainview, N.Y.; Ausubel et al., (1987) in "Current Protocols in Molecular Biology, John Wiley and Sons, New York N.Y.). Radiolabelling kits are also commercially available. The mutant tyrosinase nucleic acid sequence used as a probe in the bioassay may be RNA or DNA. Preferred methods of labelling the DNA sequences are with ³²P using Klenow enzyme or polynucleotide kinase. Preferred methods of labelling RNA or riboprobe sequences are with ³²P or ³⁵S using RNA polymerases. In addition, there are known non-radioactive techniques for signal amplification including methods for attaching chemical moieties to pyrimidine and purine rings (Dale, R. N. K. et al. (1973) Proc. Natl. Acad. Sci., 70:2238-2242; Heck, R. F. (1968) S. Am. Chem. Soc., 90:5518-5523), methods which allow detection by chemiluminescence (Barton, S. K. et al. (1992) J. Am. Chem. Soc., 114:8736-8740) and methods utilizing biotinylated nucleic acid probes (Johnson, T. K. et al. (1983) Anal. Biochem., 133:125-131; Erickson, P. F. et al. (1982) J. of Immunology Methods, 51:241-249; Matthaei, F. S. et al. (1986) Anal. Biochem., 157:123-128) and methods which allow detection by fluorescence using commercially available products. Non-radioactive labelling kits are also commercially available.

Examples of biological samples that can be used in this bioassay include, but are not limited to, primary mammalian cultures, continuous mammalian cell lines, such as melanocyte cell lines, mammalian organs such as skin or retina, tissues, biopsy specimens, neoplasms, pathology specimens, and necropsy specimens.

In another embodiment, the polynucleotides, polypeptides and variants thereof of the present invention can be used to prepare monoclonal antibodies against the soluble tyrosinase antigen. These antibodies can be used, for example, in tyrosinase detection via immunohystostaining. Therefore, the antibodies of the present invention can be used as a diagnostic reagent.

Monoclonal antibodies (MAbs) are a homogeneous population of antibodies to a particular antigen and the antibody comprises only one type of antigen binding site and binds to only one epitope on an antigenic determinant. Rodent monoclonal antibodies to specific antigens may be obtained by methods known to those skilled in the art. *See,* for example, Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991).

The present invention also discloses the use of a modified tyrosinase cDNA for the preparation of a medicament for treating melanoma. The modified tyrosinase described herein is a soluble tyrosinase mutant that is retained in the ER of a transfected/transduced antigen presenting cell(APC). This protein is then processed and the antigenic peptides derived therefrom form a complex with HLA molecules on APCs. These complexes are then recognized by cytotoxic T cells which target an abnormal cell for lysis. In a preferred embodiment, the DNA of soluble tyrosinase is delivered to professional antigen- presenting cells (e.g., dendritic cells) which will express the soluble tyrosinase and present tyrosinase antigenic peptides in the context of HLA complex. This will enhance the priming of specific CTL clones breaking tolerance against tyrosinase antigens that are presented by melanoma cells.

As discussed above, a vaccine for treating a melanoma is described herein. Vaccination can be conducted by conventional methods. For example, the immunogen can be used in a suitable diluent such as saline or water, or complete or incomplete adjuvants. Further, the immunogen may or may not be bound to a carrier to make the protein immunogenic. Examples of such carrier molecules include but are not limited to bovine serum albumin (BSA), keyhole limpet hemocyanin (KLH), tetanus toxoid, and the like. The immunogen also may be coupled with lipoproteins or administered in liposomal form or with adjuvants. The immunogen can be administered by any route appropriate for antibody production such as intravenous, intraperitoneal, intramuscular, subcutaneous, and the like. The immunogen may be administered once or at periodic intervals until a significant titer of anti-tyrosinase immune cells or anti-tyrosinase antibody is produced. The presence of anti-tyrosinase immune cells may be assessed by measuring the frequency of precursor CTL (cytoxic T-lymphocytes) against a tyrosinase antigen prior to and after immunization by a CTL precursor analysis assay (Coulie, P. et al., (1992) International Journal Of Cancer 50:289-297).

The administration of the vaccine or immunogen of the present invention may be for therapeutic purpose. The immunogen is provided at (or shortly after) the onset of the disease or at the onset of any symptom of the disease. The therapeutic administration of the immunogen serves to attenuate the disease.

By way of example, a vaccine prepared using recombinant soluble tyrosinase protein or peptide expression vectors may be used. To provide a vaccine to an individual, a genetic sequence which encodes for all or part of the soluble tyrosinase mutant nucleic acid sequence is inserted into a expression vector, as described above, and introduced into a mammal to be immunized. Examples of vectors that may be used in the aforementioned vaccines include, but are not limited to, defective retroviral vectors, adenoviral vectors, vaccinia viral vectors, fowl pox viral vectors, or other viral vectors (Mulligan, R. C., (1993) Science 260:926-932). The viral vectors carrying all or part of the soluble tyrosinase mutant nucleic sequence can be introduced into a mammal either prior to any evidence of melanoma or to mediate regression of the disease in a mammal afflicted with melanoma. Examples of methods for administering the viral vector into the mammals include, but are not limited to, exposure of cells to the virus *ex vivo,* or injection of the retrovirus or a producer cell line of the virus into the affected tissue or intravenous, administration of the virus. Alternatively the viral vector carrying all or part of the soluble tyrosinase nucleic acid sequence may be administered locally by direct injection into a melanoma lesion or topical application in a pharmaceutically acceptable carrier. Preferably, the soluble tyrosinase nucleic acids suitable for use in the present invention are provided in SEQ ID NO. 1, and variants thereof. The quantity of viral vector, carrying all or part of the mutant tyrosinase nucleic acid sequence, to be administered is based on the titer of virus particles. By way of example, a range of the immunogen to be administered is 10⁵ - 10¹³ virus particles per mammal, preferably a human.

After immunization, the efficacy of the vaccine can be assessed by production of antibodies or immune cells that recognize the antigen, as assessed by specific lytic activity, specific cytokine production, or tumor regression. One skilled in the art would know the conventional methods to assess the aforementioned parameters. If the mammal to be immunized is already afflicted with melanoma, the vaccine may be administered in conjunction with other therapeutic treatments. Examples of other therapeutic regimens includes adoptive T cell immunotherapy and coadministration of cytokines or other therapeutic drugs for melanoma.

### Method of Making

Described herein is a method for making a soluble tyrosinase mutant comprising constructing a truncated form of a human tyrosinase. In a preferred embodiment, the tyrosinase mutant has a decreased affinity for calnexin. Also preferred, the tyrosinase mutant lacks a transmembrane domain and/or is missing at least one glycosylation site. Still preferred, the tyrosinase mutant is encoded by the polynucleotide of SEQ ID No. 1 or a variant thereof.

The invention is further described by reference to the following examples, which are provided for illustration only.

### EXAMPLES

### Example 1. Materials and Methods

CHO cells (European Collection of Animal Cell Cultures, Porton Down, United Kingdom (UK)) and K42 cells (a kind gift from Dr. T. Elliott, University of Southampton and Dr.M.Michalak, University Alberta) were cultured in RPMI 1640 medium (Life Technologies, Inc., Paisley, Scotland), containing 10% fetal calf serum (FCS, Sigma, Poole, Dorset, UK), 50 units/ml penicillin, and 50 mg/ml streptomycin (Life Technologies, Inc.), and maintained at 37°C with 5% CO₂. Mouse monoclonal anti-tyrosinase antibodies (T311 antibodies) were from NeoMarkers (Fremont, USA). Rabbit polyclonal anti-calnexin antibodies were a gift from Dr. J.Bergeron (McGill University). Rabbit anti-calreticulin antibodies (calregulin C-17 antibodies) were purchased from Santa Cruz Biotechnology. NB-DNJ was a gift from Searle/Monsanto (St.Louis, MO). Radiolabeled [35S] Methionine/Cysteine was from I.C.N. Flow, (Thame, Oxfordshire, UK). CHAPS (3-[3-chloramidopropyl]-dimethylammonino-1-propanesulfate) was from Pierce Chemicals Co. Lactacystine was from Calbiochem. All other chemicals were from Sigma Chemicals Co. (St. Louis, MO).

### Example 2. Construction of a tyrosinase mutant

Full- length cDNA encoding human tyrosinase in a pcTyr cloning vector was a gift from Dr. V.J.Hearing (NCI, National Institute of Health, Bethesda, MD). WT tyrosinase cDNA and WT lumenal domain (456 aa) cDNA (ST) were amplified by PCR using pcTyr as template and the following primers:
Forward primer 5'-GCTATACCATGGCCCTCCTGGCTGTTTTG-3'
WT backward primer: 5' - GGCGCGCCTCGAGTAAATGGCTCTGATA-3'
ST backward primer: 5' - CTATTCTCGAGCCGACTCGCTTGTTC-3'

The PCR products were digested with NcoI and XhoI and cloned in frame with a 6HisTag in pTriex1 (Novagen) for mammalian expression. Sequences were confirmed by automated DNA sequencing.

### Transfection of CHO cells and metabolic labelling

CHO cells in logrithmic phase were cultured in 6-well plates for transfection and used to transiently express tyrosinase cDNA using Lipofectamine Plus (Invitrogen). Cells were harvested 24 hours after transfection and scraped and pelleted. For metabolic labelling, transfected CHO cells (10⁷ cells/ml) were starved in a cysteine-/methionine-free medium for 1 hour, pulse labelled with 100-150 µCi [³⁵S] cysteine/methionine for 20 minutes, and chased for the specified times. Immediately after chase, cells were harvested in cold PBS and incubated in 20mM N-ethylmaleimide (NEM) for 30 minutes to alkylate the free sulfhydryl groups. Cells were then lysed with CHAPS lysis buffer (50 mM HEPES buffer pH 7.5 containing 2% CHAPS, 200 mM NaCl and 0.5% protease inhibitor cocktail (Sigma) containing leupeptin, aprotinin, sodium EDTA, bestatin, AEBSF and E-64).

### Immunoprecipitation and SDS-PAGE

[³⁵S] labelled cell lysates were centrifuged and supernatants were incubated with T 311 antibodies (1: 50), or with anti-calnexin antibodies (1: 100) overnight at 4 °C. 20 µl protein A Sepharose was then added and the cell lysates were incubated for 1 hour at 4°C. The slurry was washed 3 times with 0.5% CHAPS in HEPES buffer. Tyrosinase was eluted by boiling the slurry for 5 minutes in SDS sample buffer with (reducing conditions) or without 5% 2-mercaptoethanol (non-reducing conditions). For co-immunoprecipitation studies, lysates were immunoprecipitated with anti-calnexin antibodies (1: 100) and the washed slurry was eluted with 1% SDS, diluted ten times with lysis buffer and re-precipitated with T311. Bound proteins were eluted in native or reducing conditions and resolved on a 10% SDS-PAGE gel. The gels were then visualized by autoradiography.

### DOPA Oxidase Assay

A DOPA oxidase assay measures the second catalytic activity of tyrosinase, *i.e.,* the conversion of L-DOPA to DOPAchrom via DOPA quinine. The assay was performed in gel using L-DOPA as a substrate (Negroiu *et al.,* 2000). Crude lysates or cell culture medium of transfected cells harvested 24h after transfection, were run in native conditions by SDS-PAGE and incubated in 2.5 mg/ml L-DOPA to visualize tyrosinase activity.

### Immunoblotting

Protein from the lysed CHO cells transfected with different cDNA's were electrophoretically separated in 10% acrylamide gels as described (Branza-Nichita *et al.,* 1999) and transferred to an immobilon membrane (Amersham International, Amersham, UK).

To isolate secreted tyrosinase, the culture medium was incubated with nickel-nitrilotriacetic acid - Superflow beads (Ni-NTA)(Qiagen, Chatsworth, CA) overnight at 4C. The beads were pelleted, washed three times with 20 mM imidazole and eluted with reducing SDS sample buffer. The resultant samples were separated by SDS-PAGE as above. Blots were then incubated with 1:250 dilution of anti-tyrosinase antibodies (T311) in 5% milk, 0.1 % Tween for 2 hours, at 37°C. Immunoreactivity was detected by enhanced chemiluminescent Western blotting (ECL, Amersham Corp.) according to the manufacturer's protocol.

### Example 3. Soluble tyrosinase mutant lacks enzymatic activity, accumulates in the ER and is degraded in proteasomes

Maturation of a soluble tyrosinase mutant was *monitored in vivo* by pulse-chase analysis, and immunoprecipitated with monoclonal anti-tyrosinase antibodies (T311). Samples were divided in two, and half of each sample was digested with an Endo H restriction enzyme and run next to a non-digested control in a reducing SDS-PAGE gel (Figure 1). Since Endo H digests only high-mannose and hybrid N-glycans, Endo H sensitivity was used to monitor the maturation of glycans from high-mannose to complex structures. Digestion with Endo H reduced the pool to a polypeptide that runs at 55 kD. During 5 h of chase the precursor had the same electrophoretic mobility and remained totally Endo H sensitive, indicating that its N-glycans were not processed to complex structures in the Golgi (Figure 1, lanes 1,3,5,7). A trend toward a gradual reduction in the amount of immunoprecipitated protein after 1h synthesis was observed (Figure 1).

To determine whether this was consistent with chain retention in the ER and subsequent degradation, we performed the same experiment in the presence of proteasome inhibitors. An increase in the amount of immunoprecipitated material in the presence of lactacystin relative to the untreated sample (Figure 1) was observed for the entire chase period. Endo H digestion pattern of lactacystin treated samples was similar to the untreated ones (Figure 1, lanes 9, 11, 13, 15), suggesting that ST is retained in the ER and eventually targeted for degradation in proteasomes.

To compare maturation of ST with the wild type protein, we expressed membrane tyrosinase (WT) cloned in the same vector in identical conditions (Figure 2). As shown by Endo-H digestion experiments, WT is synthesized as a 75 kD protein that acquires complex type glycans in approximately 1h of chase. The reduced ratio of complex versus high-mannose glycans reflects an overexpression of tyrosinases in this system and has been reported before (Berson *et al.,* 2000). As previously shown (Halaban *et al.,* 1997; Toyofuku *et al.,* 2001), treatment with lactacystin results in an accumulation of undegraded protein in the first 3h of the chase, suggesting that, at least in the initial stages of maturation, membrane tyrosinase is degraded in proteasomes.

The lack of processing to complex glycans displayed by the soluble form, in contrast to WT tyrosinase, suggests an incomplete maturation of the glycoprotein. This may be associated with its inability to acquire a native conformation. To address this question, we determined the enzymatic activity of ST mutant by a DOPA -oxidase assay. ST mutant is completely inactive either in the cell lysate or in the culture medium. This is in contrast to WT tyrosinase, which is able to convert the substrate DOPA to DOPA chrome. Thus, the ST chain develops into a non-native conformation devoid of biological activity.

### Example 4. Soluble and wild-type tyrosinase show different chaperone interaction patterns and folding pathways

To examine the role of calnexin and calreticulin in the folding of ST, we performed sequential immunoprecipitation experiments with anti-CNX/anti-CRT and anti-tyrosinase antibodies of metabolically labeled transfected cell lysates. In the first 30 min of chase, there was a very weak interaction of ST with both CNX (Figure 3, lanes 1, 2) and CRT (Figure 3, lanes 6, 7). Beginning with 1h-chase, the interaction became visible and increased to maximal level after 3h with both CNX (Figure 3, lanes 3-5) and CRT (Figure 3, lanes 8-9).

A different pattern was observed for WT tyrosinase, which interacted with CNX from the very early stages of folding and showed a significant decrease at 1h -chase (Figure 3, lanes 11-13). A weak interaction of CRT with WT nascent chain was evident at the end of the pulse period (Figure 3, lane 6).

To characterize the folding pathways of the WT and mutant tyrosinases, we performed immunoprecipitation experiments in transfected pulse-chased CHO cells and analyzed the samples by a non-reducing SDS-PAGE gel. These conditions allowed us to follow disulfide bond formation, which is simultaneous with a more compact conformation which results and therefore an accelerated chain mobility in the gel (Branza-Nichita *et al.,* 1999, Hebert *et al.*, 1995).

ST folds through at least three oxidation intermediates in an unproductive folding pathway as shown by a progressive increase in mobility shifts from 0 to 5h of chase (Figure 4, lanes 1-6). The first intermediate appears after pulse (0 min-chase) as compared to the reduced sample, whilst the last intermediate is observed at 3h-chase, correlating with an accelerated degradation process of truncated tyrosinase.

By analyzing WT protein folding, we could discriminate between two oxidation intermediates. The first one is not completely oxidized as shown by its similar migration velocity with the reduced sample (Figure 4, lanes 7,12). During a period of 30 min the chain is oxidized to the second intermediate (Figure 4, lane 8) that is not further oxidized. The appearance of the second intermediate correlates with the appearance of glycan complex structures and a drastic decrease in CNX interaction at 1h, indicating that the chain has acquired an export competent conformation and reached the Golgi compartment. Since the reduced ST (Figure 1, lanes 2,4,6,8) and WT pulse-chased samples (Figure 2, lanes 1-4) display uniform mobilities during the chase period, the shifts in non-reducing gels are solely due to different oxidized intermediates. In non-reducing conditions, aggregates and disulfide dimers could be seen in the early stages of folding for both ST and WT (Figure 4). These forms were absent in the last chase points and in reducing conditions, indicating the formation of mixed disulfide intermediates during folding. The data show that ST folding to a non-native conformation is six times longer than for the WT and the late oxidized intermediates are formed prior to degradation.

### Example 5. The TM domain is required for the productive folding of the chain

To investigate how folding is influenced by the presence of a transmembrane domain we have used as model a type I membrane glycoprotein-tyrosinase-and compared its folding pathway with that of a construct in which its TM domain was deleted. Tyrosinase is a melanogenic enzyme that regulates pigment synthesis in mammals (Petrescu *et αl.,* 1997). We have previously documented its folding on dependence of glycosylation (Branza-Nichita et al, 1999, 2000).

From the result of non-reducing SDS-PAGE of metabolically labelled transfected CHO cells we show that the soluble construct matures into a non-native conformation that is retained in the ER and finally degraded in proteasomes. This correlates with the absence of enzymatic activity in cells transfected with ST, as opposed to cells transfected with WT. Interestingly, the soluble form adopts an increased number of oxidized intermediates compared with WT.

The folding pathway of the two forms of tyrosinase also differ in their association pattern with CNX and CRT. The membrane-anchored chain is assisted by CNX starting from the early stages until completion of the folding process. We have previously reported a similar calnexin dependent folding for mouse membrane tyrosinase with two oxidizing intermediates occurring (Branza-Nichita *et al.,* 1999; *Branza-Nichita et al.,* 2000). By contrast, the affinity of truncated tyrosinase for CNX and CRT is initially very low and increases toward the end of the process, prior to degradation. At least two out of a total of three folding intermediates of the ST appear in the absence of CNX/CRT interaction (possibly with the assistance of other ER folding factors). These intermediates are unable to reach the native fold, implying that they have acquired aberrant disulfide bridges. Two thioreductases were shown to interact with the nascent chains during disulfide bridge formation in the ER - PDI and

Erp57 (Farmery *et al.,* 2000; *Mezghrani et al.,* 2001). Erp57 interacts with the chain when this is associated with CNX (Frickel *et al.,* 2002). It is possible that the thioreductase also catalyzes the formation of the S-S bonds in membrane tyrosinase. Conversely the oxidized intermediates of the soluble form are initially produced in the absence of CNX and Erp57 and the chain cannot be rescued to a native conformation by its late interactions with chaperones, even if both calnexin and calreticulin are shown to associate with it at this stage. There is a fragile equilibrium between folding and degradation at this stage with the quality control cycle discriminating between the correctly folded and misfolded chains. Misfolded polypeptides are re-glucosylated by GT and driven by CNX/CRT into the cycle (Sousa and Parodi, 1995). Many soluble or membrane-bound proteins have been shown to associate with CNX or CRT before being targeted to degradation (Ellgaard and Helenius, 2001).

These data indicate that re-glucosylation of misfolded ST increases in the late stages of folding and therefore, there is an increased association with both CNX and CRT-coinciding with the collapse of the chain to configurations with aberrant disulfides, just before degradation. In fact it is not the entire tyrosinase pool that is oxidized to the last intermediate; rather, some of the chains are targeted to earlier degradation. Aberrant folding and degradation of the truncated chain occurs almost simultaneously for the last two chase points. This suggests that incorrectly folded chains in the calnexin cycle are sent directly to the retro-translocation machinery. Altogether these data show that the TM domain is critical for the productive folding of tyrosinase.

The TM domain appears to play a key role in this process by increasing the time spent in the translocon region by insertion associated events and by preventing the protein from diffusing rapidly from the area. It is also worth noting that in all cases the productive folding pathways would normally include less intermediates than non-productive pathways, regardless of the length of the process. Basically, when non- - native disulfides are formed in the early stages, the chain will adopt more conformations than in the native pathway, resulting in several oxidized intermediates that will be finally targeted to degradation. Therefore an increase in the number of intermediates during folding might be an indication of a pathway leading to a non-native fold. In this case, the interaction with calnexin might be more precisely described as an early stage of degradation rather than a late stage of folding.

### Example 6. Membrane bound tyrosinase glycosylation mutants

A human tyrosinase mutant lacking the consensus sequence Asn-Arg-Thr was constructed at position 86. This was achieved by mutating Asn 86 to Gln, thereby changing the sequon to Gln-Arg-Thr. ER retention of the mutant Tyrmut1 is shown in figure 6 by its EndoH digestion pattern.

### Example 7. Membrane bound tyrosinase by anchoring through a transmembrane domain that contains ER retention signals

A tyrosinase chimeric protein (TyrE2) was constructed using the hepatitis C virus envelope protein (HCV E2) transmembrane domain and a tyrosinase ectodomain. As seen in Figure 7, the EndoH digestion of the cell lysate expressing the TyrE2 chimera resulted in a protein with an ER retention profile.

## Claims

1. A polypeptide comprising a tyrosinase mutant, wherein the tyrosinase mutant is capable of accumulating in the endoplasmic reticulum and
(a) the tyrosinase mutant is enzymatically inactive and lacks a transmembrane domain, or
(b) the tyrosinase mutant lacks a tyrosinase transmembrane domain but contains a transmembrane domain from a protein that is not tyrosinase.

2. The polypeptide of claim 1, wherein the tyrosinase mutant has a decreased affinity for calnexin.

3. The polypeptide of claim 2, wherein the tyrosinase mutant lacks a transmembrane domain.

4. The polypeptide of claim 2, wherein the tyrosinase mutant is encoded by the polynucleotide of SEQ ID No.1 or a variant thereof.

5. The polypeptide of claim 1, wherein the tyrosinase mutant is a tyrosinase chimera.

6. The polypeptide of claim 5, wherein the tyrosinase chimera is membrane bound through a transmembrane domain of another protein, and wherein the transmembrane domain contains ER retention signals.

7. The polypeptide of claim 6, wherein the tyrosinase chimera is retained in the ER through retention signals in the transmembrane domain of hepatitis C envelope protein 2.

8. An immunogenic composition comprising a tyrosinase mutant that is capable of accumulating in the endoplasmic reticulum and wherein
(a) the tyrosinase mutant is enzymatically inactive and lacks a transmembrane domain, or
(b) the tyrosinase mutant lacks a tyrosinase transmembrane domain but contains a transmembrane domain from a protein that is not tyrosinase.

9. The immunogenic composition of claim 8, wherein the tyrosinase mutant is encoded by the polynucleotide of SEQ ID No.1 or a variant thereof.

10. A polynucleotide encoding a tyrosinase mutant, wherein said tyrosinase mutant is capable of accumulating in the endoplasmic reticulum, and wherein
(a) the tyrosinase mutant is enzymatically inactive and lacks a transmembrane domain, or
(b) the tyrosinase mutant lacks a tyrosinase transmembrane domain but contains a transmembrane domain from a protein that is not tyrosinase.

11. The polynucleotide of claim 10, wherein the tyrosinase mutant lacks a transmembrane domain.

12. The polynucleotide of claim 11, wherein the tyrosinase mutant is encoded by the sequence identified in SEQ ID No. 1 or a variant thereof.

13. A vaccine comprising a polynucleotide according to claim 10 and a pharmaceutically acceptable carrier

14. The vaccine of claim 13, wherein the polynucleotide comprises the sequence identified in SEQ ID No. 1 or a variant thereof.

15. A host cell comprising a polynucleotide according to claim 10.

16. The host cell of claim 15, wherein the polynucleotide comprises the sequence set forth in SEQ ID No.1, or a variant thereof.

17. The use of a polynucleotide encoding a tyrosinase mutant for preparing a medicament for treating a melanoma, wherein said tyrosinase mutant is capable of accumulating in the endoplasmic reticulum, and wherein
(a) the tyrosinase mutant is enzymatically inactive and lacks a transmembrane domain, or
(b) the tyrosinase mutant lacks a tyrosinase transmembrane domain but contains a transmembrane domain from a protein that is not tyrosinase.

18. The use according to claim 17, wherein said medicament is administered to antigen-presenting cells.

19. The use according to claim 17, wherein said medicament elicits a cytotoxic lymphocyte immune response.

20. The use of claim 17, wherein the tyrosinase mutant accumulates in the endoplasmic reticulum of a cell.

21. The use of claim 20, wherein the tyrosinase mutant lacks a transmembrane domain.

22. A method for making a tyrosinase mutant comprising constructing a truncated form of a human tyrosinase, wherein the tyrosinase lacks a transmembrane domain and is capable of accumulating in the endoplasmic reticulum, and wherein
(a) the tyrosinase mutant is enzymatically inactive and lacks a transmembrane domain, or
(b) the tyrosinase mutant lacks a tyrosinase transmembrane domain but contains a transmembrane domain from a protein that is not tyrosinase.

23. A polypeptide comprising a human tyrosinase mutant, wherein said tyrosinase mutant is capable of accumulating in the endoplasmic reticulum, and wherein said human tyrosinase mutant also lacks the consensus sequence Asn-Arg-Thr by mutating Asn at position 86.

24. A polypeptide according to claim 23 wherein the Asn residue at position 86 is changed to a Gln residue.

## Patentansprüche

1. Polypeptid umfassend eine Tyrosinasemutante, wobei die Tyrosinasemutante in der Lage ist, sich im endoplasmatischen Reticulum anzusammeln und
(a) die Tyrosinasemutante enzymatisch inaktiv ist und ihr eine Transmembrandomäne fehlt oder
(b) der Tyrosinasemutante eine Tyrosinasetransmembrandomäne fehlt, diese jedoch eine Transmembrandomäne aus einem Protein enthält, das nicht Tyrosinase ist.

2. Polypeptid nach Anspruch 1, wobei die Tyrosinasemutante eine verminderte Affinität für Calnexin aufweist.

3. Polypeptid nach Anspruch 2, wobei der Tyrosinasemutante eine Transmembrandomäne fehlt.

4. Polypeptid nach Anspruch 2, wobei die Tyrosinasemutante durch das Polynukleotid der SEQ ID Nr. 1 oder einer Variante davon kodiert ist.

5. Polypeptid nach Anspruch 1, wobei die Tyrosinasemutante eine Tyrosinasechimäre ist.

6. Polypeptid nach Anspruch 5, wobei die Tyrosinasechimäre eine Membran ist, die durch eine Transmembrandomäne eines weiteren Proteins gebunden ist und wobei die Transmembrandomäne ER-Retentionssignale enthält.

7. Polypeptid nach Anspruch 6, wobei die Tyrosinasechimäre im ER durch Retentionssignale in der Transmembrandomäne des Hepatitis C Hüllenproteins 2 beibehalten wird.

8. Immunogene Zusammensetzung umfassend eine Tyrosinasemutante, die in der Lage ist, sich im endoplasmatischen Reticulum anzusammeln und wobei
(a) die Tyrosinasemutante enzymatisch inaktiv ist und ihr eine Transmembrandomäne fehlt oder
(b) der Tyrosinasemutante eine Tyrosinasetransmembrandomäne fehlt, diese jedoch eine Transmembrandomäne aus einem Protein enthält, das nicht Tyrosinase ist.

9. Immunogene Zusammensetzung nach Anspruch 8, wobei die Tyrosinasemutante durch das Polynukleotid der SEQ ID Nr. 1 oder einer Variante davon kodiert ist.

10. Eine Tyrosinasemutante kodierendes Polynukleotid, wobei die genannte Tyrosinasemutante in der Lage ist, sich im endoplasmatischen Reticulum anzusammeln und wobei
(a) die Tyrosinasemutante enzymatisch inaktiv ist und ihr eine Transmembrandomäne fehlt oder
(b) der Tyrosinasemutante eine Tyrosinasetransmembrandomäne fehlt, diese jedoch eine Transmembrandomäne aus einem Protein enthält, das nicht Tyrosinase ist.

11. Polynukleotid nach Anspruch 10, wobei der Tyrosinasemutante eine Transmembrandomäne fehlt.

12. Polynukleotid nach Anspruch 11, wobei die Tyrosinasemutante durch die in SEQ ID Nr. 1 identifizierte Sequenz oder eine Variante davon kodiert ist.

13. Impfstoff umfassend ein Polynukleotid nach Anspruch 10 und einen pharmazeutisch akzeptablen Träger.

14. Impfstoff nach Anspruch 13, wobei das Polynukleotid durch die in SEQ ID Nr. 1 identifizierte Sequenz oder eine Variante davon kodiert ist.

15. Wirtzelle umfassend ein Polynukleotid nach Anspruch 10.

16. wirtzelle nach Anspruch 15, wobei das Polynukleotid die in SEQ ID Nr. 1 dargelegte Sequenz oder eine Variante davon umfasst.

17. Verwendung eines Polynukleotids, das eine Tyrosinasemutante zur Herstellung eines Medikaments zur Behandlung eines Melanoms kodiert, wobei die genannte Tyrosinasemutante in der Lage ist, sich im endoplasmatischen Reticulum anzusammeln und wobei
(a) die Tyrosinasemutante enzymatisch inaktiv ist und ihr eine Transmembrandomäne fehlt oder
(b) der Tyrosinasemutante eine Tyrosinasetransmembrandomäne fehlt, diese jedoch eine Transmembrandomäne aus einem Protein enthält, das nicht Tyrosinase ist.

18. Verwendung nach Anspruch 17, wobei das genannte Medikament an antigenpräsentierende Zellen verabreicht wird.

19. Verwendung nach Anspruch 17, wobei das genannte Medikament eine zytotoxische Lymphozytenimmunantwort auslöst.

20. Verwendung nach Anspruch 17, wobei die Tyrosinasemutante sich im endoplasmatischen Reticulum einer Zelle ansammelt.

21. Verwendung nach Anspruch 20, wobei der Tyrosinasemutante eine Transmembrandomäne fehlt.

22. Verfahren zur Herstellung einer Tyrosinasemutante, umfassend die Konstruktion einer gestutzten Form einer menschlichen Tyrosinase, wobei der Tyrosinase eine Transmembrandomäne fehlt und dieses in der Lage ist, sich im endoplasmatischen Reticulum anzusammeln und wobei
(a) die Tyrosinasemutante enzymatisch inaktiv ist und ihr eine Transmembrandomäne fehlt oder
(b) der Tyrosinasemutante eine Tyrosinasetransmembrandomäne fehlt, diese jedoch eine Transmembrandomäne aus einem Protein enthält, das nicht Tyrosinase ist.

23. Polypeptid umfassend eine menschliche Tyrosinasemutante, wobei die genannte Tyrosinasemutante in der Lage ist, sich im endoplasmatischen Reticulum anzusammeln und wobei der genannten Tyrosinasemutante ebenfalls die Consensussequenz Asn-Arg-Thr durch Mutation von Asn an der Position 86 fehlt.

24. Polypeptid nach Anspruch 23, wobei der Asn-Rest an Position 86 in einen Gln-Rest umgeändert wird.

## Revendications

1. Polypeptide comprenant un mutant de tyrosinase, où le mutant de tyrosinase est capable de s'accumuler dans le réticulum endoplasmique et
(a) le mutant de tyrosinase est inactif d'un point de vue enzymatique et ne possède pas de domaine transmembranaire, ou
(b) le mutant de tyrosinase ne possède pas de domaine transmembranaire de tyrosinase mais contient un domaine transmembranaire d'une protéine qui n'est pas une tyrosinase.

2. Polypeptide selon la revendication 1, où le mutant de tyrosinase possède une affinité réduite pour la calnexine.

3. Polypeptide selon la revendication 2, où le mutant de tyrosinase ne possède pas de domaine transmembranaire.

4. Polypeptide selon la revendication 2, où le mutant de tyrosinase est codé par le polynucléotide de SEQ ID NO: 1 ou un variant de celui-ci.

5. Polypeptide selon la revendication 1, où le mutant de tyrosinase est une tyrosinase chimère.

6. Polypeptide selon la revendication 5, où la tyrosinase chimère est liée à une membrane par un domaine transmembranaire d'une autre protéine, et où le domaine transmembranaire contient des signaux de rétention dans le RE.

7. Polypeptide selon la revendication 6, où la tyrosinase chimère est retenue dans le RE par des signaux de rétention dans le domaine transmembranaire de la protéine d'enveloppe 2 de l'hépatite C.

8. Composition immunogène comprenant un mutant de tyrosinase qui est capable de s'accumuler dans le réticulum endoplasmique, et où
(a) le mutant de tyrosinase est inactif d'un point de vue enzymatique et ne possède pas de domaine transmembranaire, ou
(b) le mutant de tyrosinase ne possède pas de domaine transmembranaire de tyrosinase mais contient un domaine transmembranaire d'une protéine qui n'est pas une tyrosinase.

9. Composition immunogène selon la revendication 8, où le mutant de tyrosinase est codé par le polynucléotide de SEQ ID NO: 1 ou un variant de celui-ci.

10. Polynucléotide codant pour un mutant de tyrosinase, où ledit mutant de tyrosinase est capable de s'accumuler dans le réticulum endoplasmique, et où
(a) le mutant de tyrosinase est inactif d'un point de vue enzymatique et ne possède pas de domaine transmembranaire, ou
(b) le mutant de tyrosinase ne possède pas de domaine transmembranaire de tyrosinase mais contient un domaine transmembranaire d'une protéine qui n'est pas une tyrosinase.

11. Polynucléotide selon la revendication 10, où le mutant de tyrosinase ne possède pas de domaine transmembranaire.

12. Polynucléotide selon la revendication 11, où le mutant de tyrosinase est codé par la séquence identifiée dans SEQ ID NO: 1 ou un variant de celle-ci.

13. Vaccin comprenant un polynucléotide selon la revendication 10 et un véhicule pharmaceutiquement acceptable.

14. Vaccin selon la revendication 13, où le polynucléotide comprend la séquence identifiée dans SEQ ID NO: 1 ou un variant de celle-ci.

15. Cellule hôte comprenant un polynucléotide selon la revendication 10.

16. Cellule hôte selon la revendication 15, où le polynucléotide comprend la séquence décrite dans SEQ ID NO: 1, ou un variant de celle-ci.

17. Utilisation d'un polynucléotide codant pour un mutant de tyrosinase pour préparer un médicament destiné au traitement d'un mélanome, où ledit mutant de tyrosinase est capable de s'accumuler dans le réticulum endoplasmique, et où
(a) le mutant de tyrosinase est inactif d'un point de vue enzymatique et ne possède pas de domaine transmembranaire, ou
(b) le mutant de tyrosinase ne possède pas de domaine transmembranaire de tyrosinase mais contient un domaine transmembranaire d'une protéine qui n'est pas une tyrosinase.

18. Utilisation selon la revendication 17, où ledit médicament est administré à des cellules présentatrices d'antigène.

19. Utilisation selon la revendication 17, où ledit médicament provoque une réponse immunitaire de lymphocytes cytotoxiques.

20. Utilisation selon la revendication 17, où le mutant de tyrosinase s'accumule dans le réticulum endoplasmique d'une cellule.

21. Utilisation selon la revendication 20, où le mutant de tyrosinase ne possède pas de domaine transmembranaire.

22. Procédé pour fabriquer un mutant de tyrosinase, consistant à construire une forme tronquée d'une tyrosinase humaine, où la tyrosinase ne possède pas de domaine transmembranaire et est capable de s'accumuler dans le réticulum endoplasmique, et où
(a) le mutant de tyrosinase est inactif d'un point de vue enzymatique et ne possède pas de domaine transmembranaire, ou
(b) le mutant de tyrosinase ne possède pas de domaine transmembranaire de tyrosinase mais contient un domaine transmembranaire d'une protéine qui n'est pas une tyrosinase.

23. Polypeptide comprenant un mutant de tyrosinase humaine, où ledit mutant de tyrosinase est capable de s'accumuler dans le réticulum endoplasmique, et où ledit mutant de tyrosinase humaine ne possède également pas la séquence consensus Asn-Arg-Thr par une mutation de l'Asn à la position 86.

24. Polypeptide selon la revendication 23, où le résidu Asn à la position 86 est modifié en un résidu Gln.
